# EUROPEAN PATENT SPECIFICATION

(11) **EP 2 638 401 B1**
(45) Date of publication and mention of the grant of the patent: **15.02.2017**
(21) Application number: 11840460.7
(22) Date of filing: 14.11.2011
(51) Int. Cl.: G01N 33/68, G01N 33/53

(54) **CITRULLINATED PROTEINS: A POST-TRANSLATED MODIFICATION OF MYOCARDIAL PROTEINS AS MARKER OF PHYSIOLOGICAL AND PATHOLOGICAL DISEASE**
CITRULLINIERTE PROTEINE: POSTTRANSLATIONALE MODIFIKATION MYOKARDIALER PROTEINE ALS MARKER FÜR PHYSIOLOGISCHE UND PATHOLOGISCHE ERKRANKUNGEN
PROTÉINES CITRULLINÉES:MODIFICATION POST-TRADUCTION DE PROTÉINES MYOCARDIQUES COMME MARQUEUR DE MALADIE PHYSIOLOGIQUE ET PATHOLOGIQUE

(30) Priority: 12.11.2010 US 412819 P
(43) Date of publication of application: 18.09.2013
(73) Proprietor: The Johns Hopkins University, Baltimore, MD 21218 (US)
(72) Inventor: FERT-BOBER, Justyna P., Baltimore, Maryland 21231 (US); VAN EYK, Jennifer E., Baltimore, Maryland 21239 (US)
(74) Representative: dompatent von Kreisler Selting Werner - Partnerschaft von Patent- und Rechtsanwälten mbB
(86) International application number: PCT/US2011/060640
(87) International publication number: WO 2012/065176

(56) References cited:
- X. CHANG: "Citrullination of fibronectin in rheumatoid arthritis synovial tissue", RHEUMATOLOGY, vol. 44, no. 11, 21 June 2005 (2005-06-21) , pages 1374-1382, XP055087508, ISSN: 1462-0324, DOI: 10.1093/rheumatology/kei023
- MONIKA HERMANSSON ET AL: "Mass spectrometric analysis of rheumatoid arthritic synovial tissue identifies specific citrullination sites on fibrinogen", PROTEOMICS - CLINICAL APPLICATIONS, 25 January 2010 (2010-01-25), pages NA-NA, XP055098042, ISSN: 1862-8346, DOI: 10.1002/prca.200900088
- "Prion 2008 conference abstract book", , 8 December 2008 (2008-12-08), XP055098320, Retrieved from the Internet: URL:http://www.neuroprion.org/resources/pd f_docs/conferences/prion2008/abstract-book -prion2008.pdf [retrieved on 2014-01-24]
- CHANG XIAOTIAN ET AL: "Increased PADI4 expression in blood and tissues of patients with malignant tumors", BMC CANCER, BIOMED CENTRAL, LONDON, GB, vol. 9, no. 1, 30 January 2009 (2009-01-30), page 40, XP021049049, ISSN: 1471-2407, DOI: 10.1186/1471-2407-9-40
- PAM R. TAUB ET AL: "Biomarkers of Heart Failure", CONGESTIVE HEART FAILURE, vol. 16, 23 July 2010 (2010-07-23), pages S19-S24, XP055098326, ISSN: 1527-5299, DOI: 10.1111/j.1751-7133.2010.00168.x

### SEQUENCE LISTING

<110> THE JOHNS HOPKINS UNIVERSITY
<120> CITRULLINATED PROTEINS: A POST-TRANSLATED MODIFICATION OF MYOCARDIAL PROTEINS AS MARKER OF PHYSIOLOGICAL AND PATHOLOGICAL DISEASE
<130> 2240.320309
<140>
   <141>
<150> 61/412,819
   <151> 2010-11-12
<160> 34
<170> PatentIn version 3.5
<210> 1
   <211> 9
   <212> PRT
   <213> Homo sapiens
<400> 1
   Arg Ser Leu Ser Thr Glu Leu Phe Lys
   1               5
<210> 2
   <211> 19
   <212> PRT
   <213> Homo sapiens
<400> 2
   Arg Asp Leu Glu Glu Ala Thr Leu Gln His Glu Ala Thr Ala Ala Ala
   1               5                   10                  15
   Leu Arg Lys
<210> 3
   <211> 17
   <212> PRT
   <213> Homo sapiens
<400> 3
   His Arg Leu Gln Asn Glu Ile Glu Asp Leu Met Val Asp Val Glu Arg
   1               5                   10                  15
   Ser
<210> 4
   <211> 15
   <212> PRT
   <213> Homo sapiens
<400> 4
   Lys Lys Leu Ala Gln Arg Leu Gln Glu Ala Glu Glu His Val Glu
   1               5                   10                  15
<210> 5
   <211> 20
   <212> PRT
   <213> Homo sapiens
<400> 5
   Arg Pro Ala Pro Asp Ala Pro Glu Asp Thr Gly Asp Ser Asp Glu Trp
   1               5                   10                  15
   Val Phe Asp Lys
               20
<210> 6
   <211> 9
   <212> PRT
   <213> Homo sapiens
<400> 6
   Glu Asp Arg Tyr Glu Glu Glu Ile Lys
   1               5
<210> 7
   <211> 16
   <212> PRT
   <213> Homo sapiens
<400> 7
   Glu Thr Arg Ala Glu Phe Ala Glu Arg Ser Val Thr Lys Leu Glu Lys
   1               5                   10                  15
<210> 8
   <211> 14
   <212> PRT
   <213> Homo sapiens
<400> 8
   Ala Glu Glu Ala Asp Arg Lys Tyr Glu Glu Val Ala Arg Lys
   1               5                   10
<210> 9
   <211> 23
   <212> PRT
   <213> Homo sapiens
<400> 9
   Ala Gly Phe Ala Gly Asp Asp Ala Pro Arg Ala Val Phe Pro Ser Ile
   1               5                   10                  15
   Val Gly Arg Pro Arg His Gln
               20
<210> 10
   <211> 10
   <212> PRT
   <213> Homo sapiens
<400> 10
   Val Asn Ser Arg Pro Ile Lys Asp Leu Lys
   1               5                   10
<210> 11
   <211> 11
   <212> PRT
   <213> Homo sapiens
<400> 11
   Tyr Arg Ile Gln Glu Phe Lys Gly Gly Tyr His
   1               5                   10
<210> 12
   <211> 16
   <212> PRT
   <213> Homo sapiens
<400> 12
   Asp Ile Leu Ile Pro Pro Glu Gly Glu Leu Asp Ala Asp Leu Arg Lys
   1               5                   10                  15
<210> 13
   <211> 15
   <212> PRT
   <213> Homo sapiens
<400> 13
   Val Ile Ala Glu Arg Gly Leu Gly Asp Val Asp Gln Leu Val Lys
   1               5                   10                  15
<210> 14
   <211> 20
   <212> PRT
   <213> Homo sapiens
<400> 14
   Ile Val Val Val Thr Ala Gly Val Arg Gln Gln Glu Gly Glu Ser Arg
   1               5                   10                  15
   Leu Asn Leu Val
               20
<210> 15
   <211> 22
   <212> PRT
   <213> Homo sapiens
<400> 15
   Arg Leu Tyr Gly Ser Glu Ala Phe Ala Thr Asp Phe Gln Asp Ser Ala
   1               5                   10                  15
   Ala Ala Lys Lys Leu Ile
   20
<210> 16
   <211> 14
   <212> PRT
   <213> Homo sapiens
<400> 16
   Ser Arg Asp Leu Gln Leu Ala Val Asp Gln Leu Lys Leu Lys
   1 5 10
<210> 17
   <211> 13
   <212> PRT
   <213> Homo sapiens
<400> 17
   Lys Leu Asp Pro Leu Glu Gly Leu Asp Glu Pro Thr Arg
   1 5 10
<210> 18
   <211> 23
   <212> PRT
   <213> Homo sapiens
<400> 18
   Arg Gln Pro Pro Ala Ala Leu Ala Lys Pro Pro Gly Thr Pro Pro Ser
   1 5 10 15
   Leu Gly Ala Ser Pro Ala Lys
   20
<210> 19
   <211> 9
   <212> PRT
   <213> Homo sapiens
<400> 19
   Arg Ser Leu Ser Thr Glu Leu Phe Lys
   1 5
<210> 20
   <211> 20
   <212> PRT
   <213> Homo sapiens
<400> 20
   Leu Ile Ser Gln Leu Thr Arg Gly Lys Leu Thr Tyr Thr Gln Gln Leu
   1               5                   10                  15
   Glu Asp Leu Lys
               20
<210> 21
   <211> 16
   <212> PRT
   <213> Homo sapiens
<400> 21
   Gln Arg Leu Gln Asp Ser Glu Glu Gln Val Glu Ala Val Asn Ala Lys
   1               5                   10                  15
<210> 22
   <211> 20
   <212> PRT
   <213> Homo sapiens
<400> 22
   Arg Pro Ala Pro Asp Ala Pro Glu Asp Thr Gly Asp Ser Asp Glu Trp
   1               5                   10                  15
   Val Phe Asp Lys
               20
<210> 23
   <211> 16
   <212> PRT
   <213> Homo sapiens
<400> 23
   Glu Thr Arg Ala Glu Phe Ala Glu Arg Ser Val Thr Lys Leu Glu Lys
   1               5                   10                  15
<210> 24
   <211> 24
   <212> PRT
   <213> Homo sapiens
<400> 24
   Leu Glu Glu Ala Glu Lys Ala Ala Asp Glu Ser Glu Arg Gly Met Lys
   1               5                   10                  15
   Val Ile Glu Asn Arg Ala Leu Lys
               20
<210> 25
   <211> 23
   <212> PRT
   <213> Homo sapiens
<400> 25
   Ala Gly Phe Ala Gly Asp Asp Ala Pro Arg Ala Val Phe Pro Ser Ile
   1               5                   10                  15
   Val Gly Arg Pro Arg His Gln
               20
<210> 26
   <211> 15
   <212> PRT
   <213> Homo sapiens
<400> 26
   Val Ile Ala Glu Arg Gly Leu Gly Asp Val Asp Gln Leu Val Lys
   1               5                   10                  15
<210> 27
   <211> 9
   <212> PRT
   <213> Homo sapiens
<400> 27
   Leu Phe Pro Gly Arg Pro Pro Val Lys
   1               5
<210> 28
   <211> 15
   <212> PRT
   <213> Homo sapiens
<400> 28
   Asn Asp Glu Arg Asn Tyr Arg Glu Ile Pro Ala Ile Lys Ile Lys
   1               5                   10                  15
<210> 29
   <211> 21
   <212> PRT
   <213> Homo sapiens
<400> 29
   Cys Asp Val Phe Met Arg Cys Arg Leu Val Asp Ala Asp Gly Pro Leu
   1               5                   10                  15
   Ala Arg Leu Lys Lys
               20
<210> 30
   <211> 11
   <212> PRT
   <213> Homo sapiens
<400> 30
   Tyr Arg Ile Gln Glu Phe Lys Gly Gly Tyr His
   1 5 10
<210> 31
   <211> 14
   <212> PRT
   <213> Homo sapiens
<400> 31
   Leu Ser Gly Val Leu Thr Val Lys Ala Gly Asp Thr Ile Arg
   1 5 10
<210> 32
   <211> 13
   <212> PRT
   <213> Homo sapiens
<400> 32
   Lys Leu Asp Pro Leu Glu Gly Leu Asp Glu Pro Thr Arg
   1 5 10
<210> 33
   <211> 21
   <212> PRT
   <213> Homo sapiens
<400> 33
   Phe Tyr Gln Thr Ser Val Glu Ser Thr Asp Phe Ala Asn Ala Pro Glu
   1 5 10 15
   Glu Ser Arg Lys Lys
   20
<210> 34
   <211> 25
   <212> PRT
   <213> Homo sapiens
<400> 34
   Gly Gln Glu Lys Thr Cys Arg Glu Thr Glu Val Gly Asp Pro Ala Gly
   1 5 10 15
   Asn Glu Leu Ala Glu Pro Glu Ala Lys
   20 25

## Description

This invention was made with Government support of an NHLBI Proteomic Contract No. NIH N01-HV-28180 and RO1HL63038, awarded by the National Institutes of Health. The Government has certain rights in this invention.

### BACKGROUND

Despite tremendous advances in cardiovascular research and clinical therapy, heart disease remains the leading cause of morbidity and mortality in western society and is growing in developing countries. Cardiovascular diseases (CVD) have been subjected to extensive study, covering all major pathological conditions: including ischemic heart disease (IHD) and heart failure (HF) the two primary causes of CVD. Heart failure is characterized by reduced blood supply to the heart muscle with resulting decreased function. It is also appreciated that treating heart failure patients with drugs (such as cardiac glycosides) augment pump function by increasing the contractility of cardiac myocytes and can improve hemodynamics and exercise tolerance. These observations led to the "hemodynamic hypothesis" that heart failure is primarily caused by defective cardiac myocyte contractility. It is important to point out that other factors - changes in cardiac structure (dilation), cell death (apoptosis), altered vascular structure and reactivity, abnormal energy utilization, and neurohormonal disturbances also contribute to the progression of CVD. Cardiac contractile function is, in part, regulated by post-translational modifications (PTMs) to the myofilament. This machinery is directly responsible for the force-generating process. Both dynamic and irreversible PTMs, like phosphorylation, occur to myofilament proteins and have been observed in numerous models of heart disease.

Importantly, IHD can occur acutely, resulting in myocardial stunning or myocardial infarction (heart attack) or chronically which is one common cause of heart failure. Interestingly, in the acute situation, intermittent ischemia events can be protective against a subsequently more severe ischemic event reducing cell death and injury to the heart. This is termed myocardial preconditioning and is known to occur to many other organs, including kidney and skeletal muscle. Preconditioning, in part reduces the drop in cellular pH and increase in calcium concentration that occurs with reperfusion. This condition can effect the peptidyl arginine deiminases (PADs) which are a family of calcium dependent enzymes that post-translationally convert arginine residues on substrate proteins to the non-standard amino acid citrulline.

The enzymatic conversion of arginine into citrulline occurs in physiological processes such as epidermal differentiation, formation of the hair follicle and differentiation of the myelin sheath during development of the central nervous system. It was first linked to human pathology by the demonstration of citrullinated proteins in the synovium of patients with rheumatoid arthritis. More recently, protein citrullination has been described in non-rheumatoid inflammatory synovitis and also in autoimmune neurodegenerative diseases such as multiple sclerosis and Alzheimer's disease. In light of these observations, we asked whether citrullination occurs in the heart and whether this modification will provide insights into the pathologies of specific disease states in cardiac. Furthermore, there have been no investigations to determine if PADs are present in the heart during health or disease events.

The incident rates of heart failure and diastolic dysfunction are increased in rheumatoid arthritis (RA) patients compared to non-RA controls, suggesting that myocardial remodeling occurs as part of the RA disease process. The phenotype of heart failure in RA differs from that of non-RA patients, characterized by fewer symptoms, lower blood pressure, and higher ejection fraction at presentation, suggesting that the pathophysiologic mechanisms underlying the progression to heart failure in RA patients may be different from those of the general population. Recently, it was reported that an association of higher concentration of serum anti-CCP antibodies with lower myocardial mass and smaller left ventricular chamber volume in RA patients without known cardiovascular disease; raising the possibility that RA-specific autoimmunity against citrullinated proteins might mediate changes to myocardial morphology that, in turn, may affect myocardial function. Citrullination, the post-translational modification of basic amino acid arginine to neutral amino acid citrulline results in basic charge loss which can influence the overall charge distribution, isoelectric point, as well as the ionic and hydrogen bond formation. This PTM is crucial as it can alter the physical and chemical properties of proteins, regulating protein folding, distribution, stability, activity and function. The reaction is catalyzed by a set of peptidyl arginine deiminase enzymes (PADs), that are abundant in the rheumatoid synovium but not restricted to RA.
In North America, infectious-cardiomyopathy can occur during or follow viral infections (e.g. coxsackievirus B3, adenoviruses or parvovirus B 19). In fact, immunocardiomyopathy is an important cause of HF or sudden death especially in children and young adults. Therefore, we believe that myocardial citrullination would be more abundant in RA compared to other conditions, and that myocardial regions demonstrating citrullination would co-localize with evidence of tissue damage (i.e. myocarditis, fibrosis, etc.) and PADs.
Taub et al., Congestive Heart Failure 16 (2010) 19-24 provide an overview of biomarkers of heart failure.

### SUMMARY

In an embodiment, the invention is directed to a method of diagnosing certain cardiovascular diseases in a subject comprising detecting the presence of a citrullinated protein in a biological sample obtained from a subject as defined in claims 1 and 2.

### BRIEF DESCRIPTION OF THE DRAWINGS

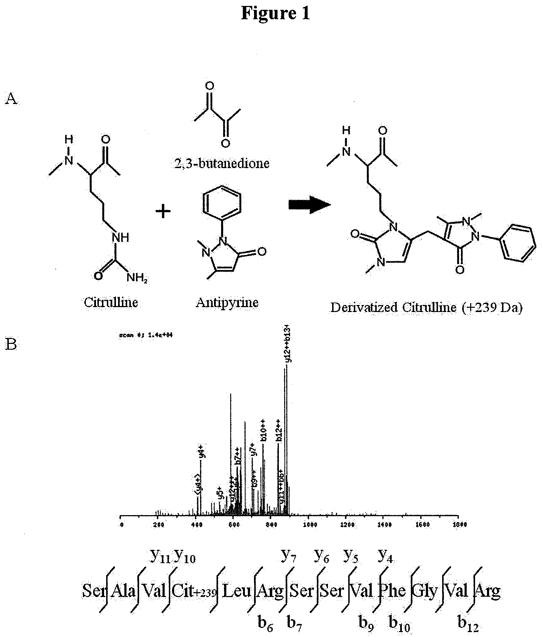

FIG. 1: Citrullination derivation scheme and its detection by mass spectroscopy. A) The ureido group of citrulline is modified by 2,3-butanedione and antipyrine to form a modified citrulline residue. The mass is increased by 239 Da. B) MS/MS spectrum illustrating the identification of the derivatized citrullines.

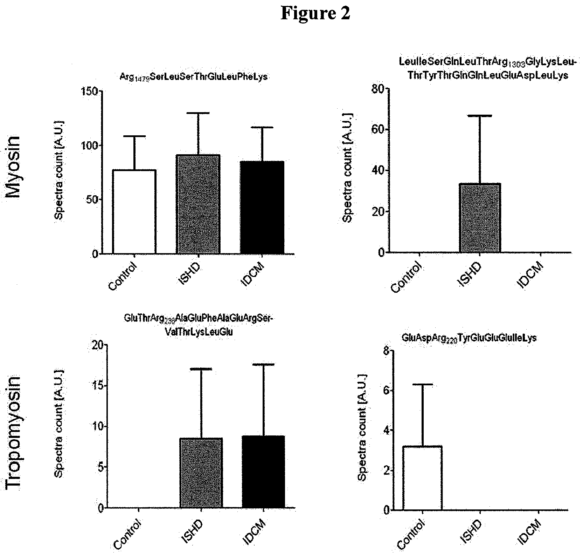

FIG. 2: Site specific endogenous citrullination of control versus HF samples (IHD, IDCM). Myosin heavy chain has four citrullinated sites; tropomysin also has four citrullinated sites; however there is a difference between the site specificity of modified residues.

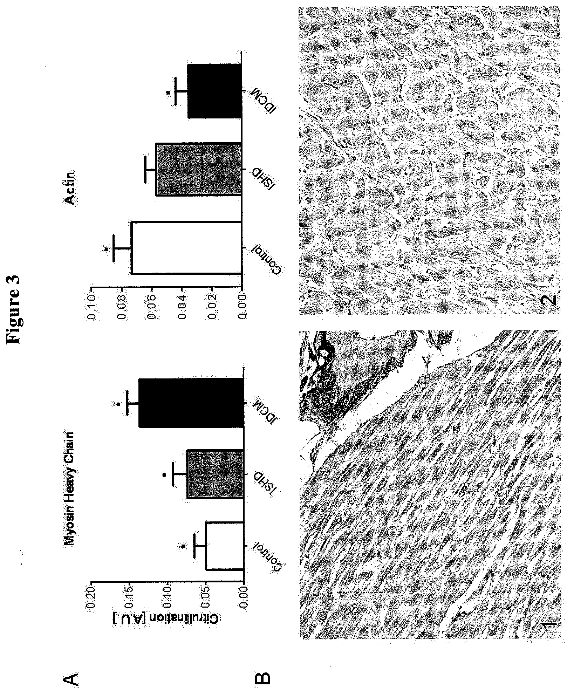

FIG. 3: A) Detection of citrullinated proteins in heart homogenate obtained from control and HF patients (IHD, IDCM). Citrullination of myofilament proteins was expressed relative to direct blue staining to correct for differences in protein loading. *p<0.05 donor vs. IHD vs. IDCM in t-test. IN IDCM tissue, a significant increase was seen in myosin heavy chain citrullination vs. control and IHD. A small decrease of actin citrullination was observed in donor tissue vs. IDCM samples. B) Immunohistochemical staining of citrullinated proteins in heart from control patients. (1) Citrullinated proteins were detected in the myrofibrils of control. (2) In the negative control the myofibrils are clearly unstained.

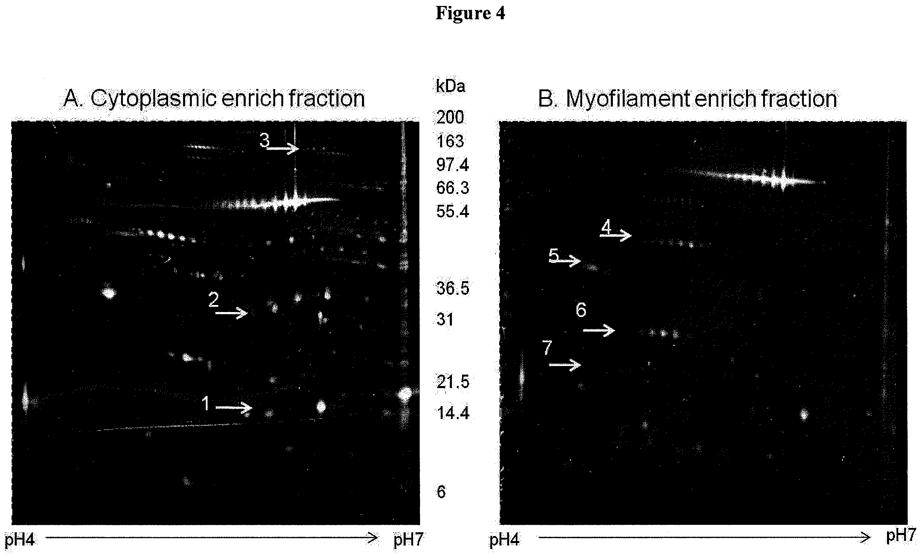

FIG. 4: Verification of the high abundant proteins was carried out with 2D-DIGE analysis of control and PAD2 treated human heart (left ventricle). Samples were labeled with Cy2 (internal control), Cy3 (untreated) and Cy5 (treated). Each gel contains 150 µg of total protein separated by pI ranges 4-7 in the first dimension and 10% linear polyacrylamide gel in the 2D. A) Representative large 2D-DIGE gel of cytoplasmic and B myofilament enrich fraction with green shift indicating citrullination. Relevant images were captured by excitation with different laser using Typhoon 9210. The arrows indicate differentially regulated protein spots determined by image analysis and identified by LC-MS/MS. Proteins are numbered according to Table 2.

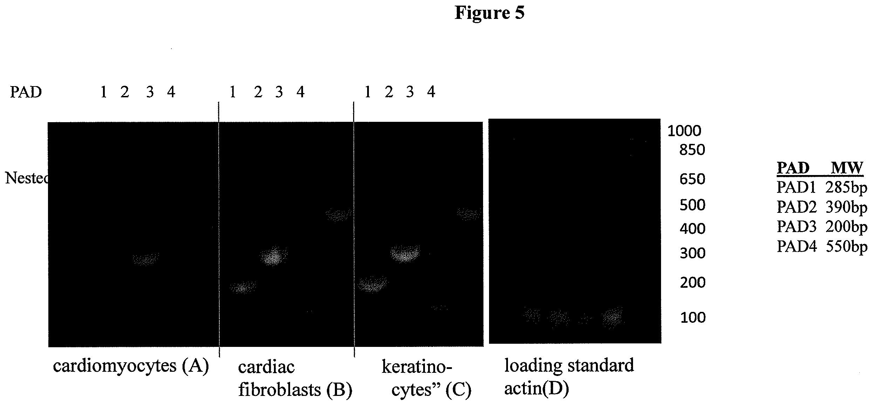

FIG 5: RT-PCR analysis of expression level of PAD isoforms in heart from control mouse (A, B) mouse keratinocytes (C). The PCR products PAD2 is seen in all types of samples; PAD 4 and PAD1 is seen in cardiac fibroblast and keratinocytes. PAD3 has not been seen in any of the samples.

### DETAILED DESCRIPTION

Post-translational modification (PMT) of arginine to citrulline by peptidylarginine deiminases (PADs) is abundant in rheumatoid synovium, and autoimmunity against citrullinated proteins is highly specific for RA (Giles et al. Arthritis Rheum 2010; 62:940-951) and a strong predictor of articular damage (van Gaalen et al. Arthritis Rheum 2004; 50(7):2113-2121). Citrullination is observed in tissues other than rheumatoid synovium, and typically in conditions characterized by inflammation/autoimmunity, such as multiple sclerosis, inflammatory bowel disease, and polymyositis (Makrygiannakis et al. Ann Rheum Dis 2006; 9:1219-1222) ischemic heart disease (IHD) and heart failure (HF). While it is unclear whether immune targeting of citrullinated proteins mediates any of the phenotypic features of these disorders, the abundant citrullination observed in these varied disorders suggests that other tissues, such as the myocardium, may also demonstrate post-translational citrullination.

Protein citrullination is catalyzed by a family of Ca²⁺ dependent enzymes, peptidyl arginine deiminases (PADs), which deiminate positively charged arginine residues to neutral citrulline which can change the structure and function of a protein due to the loss of the basic character. This post-translational modification (PTM) has become an area of interest due to its role in several physiological and pathological processes. Physiological processes include epithelial terminal differentiation, gene expression regulation, and apoptosis. Multiple sclerosis, Alzheimer's disease and Rheumatoid arthritis (RA) are examples of human diseases where protein citrullination involvement has been demonstrated. We propose that protein citrullination plays a role in the progression and development of ischemia/reperfusion injury or heart failure (HF) alone or in terms of RA and other diseases. Additionally some of the protein targets that undergo citrullination in rheumatoid synovium (i.e. vimentin, enolase, fibronectin) and are targets for anti-CCP antibodies in RA are also present in the myocardium (Giles et al., Arthritis Rheum 2010, 62:940-951). We show that proteins in the myocardium serve as substrates for citrullination. Furthermore, and not to be limited to a particular mechanism of dysfunction, it is possible that citrullination of the fundamental contractile element in the myofilament could contribute to myocardial dysfunction. We show that protein citrullination occurs in normal hearts (healthy) and that the protein citrullination status changes with heart disease. We have identified citrullinated proteins and their modified amino acid residues in tissue isolated from the heart as well as from cardiac myocytes. The number of modified proteins and the modified amino acids can reflect different heart disease phenotypes. In one embodiment, the invention focuses on the detection of the citrullinated proteins and sites of citrullination of the intact or degraded protein in plasma or serum as well as tissue (e.g. biopsy). In another embodiment, the citrullinated proteins can be used as a diagnostic marker for myocardial disease. In another embodiment, PAD activity may be modulated.

Regulation and augmentation of myocardial contractility is required in many disease settings. Citrullination of key cardiac specific myofilament proteins occur and thus, could have a pivotal role in regulating the contractile activity of the heart. Regulation (increase or decrease) of citrullination could allow control of heart function in acute (such as in ischemia reperfusion injury) and chronic (such as failing heart) disease phenotypes. Detection of citrullinated proteins in tissue or body fluids (eg., blood, plasma and serum) can be a biomarker(s) for diagnosis, prognosis or risk stratification in patients with cardiac disease including myocardial injury and heart failure. The citrullinated cardiac proteins may act as immune targets for circulating autoantibodies, especially if secreted or released following myocardial injury.

Role of cardiomyopathy in RA: Increased evidence of cardiovascular morbidity and mortality in RA patients has been only recently recognized. Studies have shown that the risk of myocardial infarction (MI), heart failure (HF) and stroke is higher in patients with RA and can cause up to 40% of deaths in these patients (Wolfe et al., Arthritis Rheum 2008, 9:2612-2621; Levy et al., Clin Exp Rheumatol 2008, 4:673-679; Nadareishvili et al., Arthritis Rheum 2008, 8:1090-1096; Lopex-Longo et al., Arthritis Rheum 2009 4:419-424; Sihvonen et al. Scand J Rheumatol 2004 33:221-227). It is speculated in rheumatoid arthritis that the underlying inflammatory processes of the disease contributes to production/induction of anti-CCP antibodies, which precedes the onset of RA, and can be independently associated with the development of ischemic heart disease (Turesson et al., Ann Rheum Dis 2007, 66:70-75). Anti-CCP antibodies are specific markers for RA (Yamada et al., Future Rheumatology 2006, 2:249-258). Citrullination also occurs in autoimmune neurodegenerative diseases such as multiple sclerosis and Alzheimer's disease (Nicholas et al., J Comp Neurol 2003,2:51-66; Shida-Yamamoto et al., Journal of Investigative Dermatology 2002, 118:282-287) as well as in various general biological processes such as epithelial terminal differentiation, gene expression regulation, and apoptosis (Shibata et al., Journal of Dermatological Science 2009, 53:34-39; Mastronardi et al., JNeurosci 2006, 44:11387-11396; Lundberg et al., Arthritis Rheum 2008, 58:3009-3019; Raptopoulou et al., Crit Rev Clin Lab Sci 2007, 44:339-363; Gabriel et al., Arthritis Rheum 1999, 42:415-420). We investigated fractions of human heart and determined the citrullinated protein in healthy vs. HF individuals. Furthermore we determined whether the proteins that have modification(s) change with disease or if modification can have an influence on protein function.

Citrullination (also known as deimination) is a PTM (posttranslational modification) characterized by the conversion of a positively charged amino acid residue, arginine, to a neutrally charged citrulline (Figure 1). Introduction of citrulline can dramatically change the structure and function of a protein due to the loss of the strong basic character (pI=10.76) which influences the overall charge distribution, isoelectric point, and the ionic and hydrogen bond forming abilities of the protein. While not wishing to be limited to theory, such changes may alter the protein structure and results in a somewhat looser, more open configuration (Tarcsa et al., J Biol Chem 1996, 48:30709-30716). Therefore, citrullination may also influence the interaction of the molecule with other proteins. For example, citrullination of vimentin filaments can induce almost complete depolymerization, disrupting the cell's cytoskeletal network (Inagaki et al., J Biol Chem 1989, 264:18119-18127; Backs et al., Circulation Research 2006, 98:15-24). In histones, citrullination was recently found to have a repressive effect in nucleosome-nucleosome interactions, which consequently affects higher-order chromatin structure (Spencer et al., Gene 1999, 240:1-12). Recent work has demonstrated the importance of chromatin remodeling in the control of cardiac growth and gene expression in response to acute and chronic stress stimuli. Additionally, arginine methylation in histones also affects chromatin structure. Importantly for our study, it has been shown that the enzymes utilized in citrullination can indirectly antagonize arginine methylation (Spencer et al., Gene 1999, 240:1-12). This functional connection between citrullination and deacetylation of histones may have some undercover implication in remodeling or gene expression in response to acute and chronic stress stimuli leading to altered cardiac function.

Herein, the inventors disclose citrullinated proteins in healthy patients (Table 1A) and in heart failure (diseased) patients (Table 1B). Citrullination occurs in specific proteins, including the myofilament proteins; tropomyosin, myosin (heavy and light chain) and myosin binding protein C, suggesting that this modification could also be seen in skeletal muscle, which is also predominated by these myofilament proteins. With this information, it is now possible to diagnose susceptibility to cardiovascular disease and susceptibility to autoimmunity to citrullinated proteins. Accordingly, methods of diagnosing cardiovascular disease are disclosed. Methods of diagnosing susceptibility to autoimmunity to citrullinated proteins in cardiovascular disease are disclosed. Either method includes the detection of citrullinated proteins in tissue or body fluids, including blood, plasma or serum.

**Table 1A. List of citrullinated protein in healthy individuals identified by the targeted analysis using MS/MS (tandem mass spectrometry)**

| Protein ID | Modifiedresidue in sequence | Protein function |
|---|---|---|
| **Myosin heavy chain** | Arg₁₄₇₉SerLeuSerThrGluLeuPheLys (SEQ ID NO: 1) | Muscle contraction. |
| | | |
| | | |
| | | |
| **Myosin binding protein C, cardiac** | | Muscle contraction and hold the thick filament together. |
| **Tropomyosin α 1** | GluAspArg₂₂₀TyrGluGluGlullelys (SEQ ID NO: 6) | Plays a central role in the calcium dependent regulation of muscle contraction |
| | | |
| **Tropomyosin α 3** | | Plays a role, in association with the troponin complex, in regulation of vertebrate striated muscle contraction |
| **Actin** | | Stretch and contractility activity. |
| **Titin** | ValAsnSerArg₁₅₁₇₂ProlleLysAspLeuLys (SEQ ID NO: 10) | Key component in the assembly and functioning of vertebrate striated muscles; it contributes to the fine balance of forces between the two halves of the sarcomere. |
| | TyrArg₃₁₈₁₁lleGlnGluPheLysGlyGlyTyrHis (SEQ ID NO: 11) | |
| | | |
| **Lipoprotein lipase** | | Hydrolysis of triglycerides of circulating chylomicrons and very low density lipoproteins (VLDL). Binding to heparin sulfate proteogylcans at the cell surface is vital to the function. |
| **L-lactate dehydrogenase B chain** | | Catalytic activity |
| | | (S)-lactate + NAD⁺ = pyruvate + NADH. |
| **Alpha-1-antichymotrypsin** | | Can inhibit neutrophil cathepsin G and mast cell chymase, both of which can convert angiotensin-1 to the active angiotensin-2. |
| **Caspase recruitment domain-containing protein 10** | | Activates NF-kappa-B via BCL10 and IKK. |
| **Zinc finger ZZ-type and EF-hand domain-containing protein 1** | | Protein binding |
| **Caskin 1** | | May link the scaffolding protein CASK to downstream intracellular effectors |

**Table IB. List of citrullinated proteins found in HF patients by the targeted analysis using MS/MS**

| Protein ID | Modified residue in sequence | Protein function |
|---|---|---|
| **Myosin heavy chain** | Arg₁₄₇₉SerLeuSerThrGluLeuPheLys (SEQ ID NO: 19) | Muscle contraction. |
| | | |
| | | |
| **Myosin binding protein C, cardiac** | | Muscle contraction and hold the thick filament together. |
| **Tropomyosin α 1** | | Plays a central role in the calcium dependent regulation of muscle contraction |
| **Tropomyosin 3** | | Binds to actin filaments, regulates of vertebrate striated muscle contraction |
| **Actin** | | Stretch and contractility activity. |
| **Lipoprotein lipase** | | Hydrolysis of triglycerides of circulating chylomicrons and very low density lipoproteins (VLDL). Binding to heparin sulfate proteogylcans at the cell surface is vital to the function. |
| **Sulfhydryl oxidase 2** | LeuPheProGlyArg₃₆₇ProProValLys (SEQ ID NO: 27) | Catalyzes the oxidation of sulfhydryl groups to disulfides with the reduction of oxygen to hydrogen peroxide. |
| **Putative zinc finger protein 818** | | May be involved in transcriptional regulation. |
| **Disintegrin and metalloproteinase domain-containing protein 10** | | Responsible for the proteolytic release of TNF-alpha and other cell-surface proteins, including heparin-binding epidermal growth-like factor, ephrin-A2 |
| **Titin** | TyrArg₃₁₈₁₁lleGlnGluPheLysGlyGlyTyrHis (SEQ ID NO: 30) | Key component in the assembly and functioning of vertebrate striated muscles; it contributes to the |
| | LeuSerGlyValLeuThrValLysAlaGlyAspThrlleArg₁₉₀₅₅ (SEQ ID NO: 31) | fine balance of forces between the two halves of the sarcomere. |
| **Zinc finger ZZ-type and EF-hand domain-containing protein 1** | LysLeuAspProLeuGluGlyLeuAspGluProThrArg₂₄₆₄ (SEQ ID NO: 32) | Protein binding |
| **Serpin** | | Act as a protease inhibitor to modulate the host immune response against tumor cells. |
| **tRNA-dihydrouridine synthase** | | Catalyzes the synthesis of dihydrouridine, a modified base found in the D-loop of most tRNAs |

The conversion of arginine amino acid residues to citrulline has several implications for the structure of a protein; for example, the ureido group of citrulline may have a destabilizing effect on protein structure due to its urea-like properties; it may also provoke a conformational change, and may alter the isoelectric point (pI) value and electrophoretic mobility. This destabilizing effect of citrulline has been described on several proteins including filaggrin, trichohyalin and myelin basic protein resulting in a loss of the organized secondary structure of these proteins. Loss of the positive charge associated with citrullination can also be expected to have a large impact on interactions between proteins, modulate signaling potency and interfere with susceptibility to proteolytic degradation. Therefore, this PTM has become an area of interest due to its role in several physiological and pathological processes. Physiological processes include epithelial terminal differentiation, gene expression regulation, and apoptosis. Multiple sclerosis, Alzheimer's disease and Rheumatoid arthritis (RA) are examples of human diseases where protein citrullination involvement has been demonstrated. Protein citrullination may play a role in the progression and development of ischemia/reperfusion injury, myocardial preconditioning or heart failure.

There are five PAD isoforms (PAD1 to PADS4 and PAD6), each encoded by a separate gene. Although the isoforms share a high degree of amino acid sequence homology, they appear to have different tissue-specific expression. RT-PCR or Northern blot analysis revealed that PAD 2 and PAD4 are found in isolated cardiac myocytes (adult) and additionally PAD1 in isolated cardiac fibroblasts (adult) obtained from the heart. PAD1, PAD2 and PAD4 are also present in skeletal muscle but at a higher level than in the heart. This finding suggests that these isoforms are responsible for the deamination of arginine in the heart. As disclosed herein, modulation of the PAD isoforms which are specific to cardiac myocytes and cardiac fibroblasts is possible using interfering RNA (e.g. siRNA) developed against PAD or against a specific isoform. Gene therapy approaches can be used to increase PAD isoforms in target tissues. This can be accomplished using a modified virus or other well established methods for *in vivo* incorporation of DNA. Specific inhibitors of PAD isoforms can be used to reduce endogenous activity. Further, mutation of the Arg and Cys within the catalytic domain of PAD to Ala or another amino acid residue can be used to reduce enzyme function.

There are a number of PAD inhibitors known in the art which are useful in a method to modulate PAD isoform activity. These include F-amidine [N-α-benzoyl-N5-(2-fl uoro-1-iminoethyl)-1-ornithine amide], 2-chloroacetamidine and Cl-amidine[N-α-benzoyl-N5-(2-chloro-1-iminoethyl)-1-ornithine amide].

### Definitions

The following terms are used as defined below throughout this application, unless otherwise indicated.

"Marker" or "biomarker" are used interchangeably herein, and in the context of the present invention refer to a protein or peptide that has specific citrullinated amino acid residues or the enzyme itself, PAD 1, PAD2 or PAD4 (of a particular specific identity or apparent molecular weight) which is differentially present in a sample taken from patients having a specific disease or disorder as compared to a control value, the control value consisting of, for example, average or mean values in comparable samples taken from control subjects (e.g., a person with a negative diagnosis, normal or healthy subject). Biomarkers may be determined as specific peptides or proteins (Table 1A or Table 1B) which may be detected by antibodies or mass spectroscopy. In some applications, for example, a mass spectroscopy or other profile or multiple antibodies may be used to determine multiple biomarkers, and differences between individual biomarkers and/or the partial or complete profile may be used for diagnosis. This can include detection of the enzyme or a protein it has citrullinated, alone or in combination.

The phrase "differentially present" refers to differences in the quantity and/or the frequency of a marker present in a sample taken from patients having a specific disease or disorder as compared to a control subject. For example, a marker can be present at an elevated level or at a decreased level in samples of patients with the disease or disorder compared to a control value (e.g. determineed from samples of control subjects). Alternatively, a marker can be detected at a higher frequency or at a lower frequency in samples of patients compared to samples of control subjects. A marker can be differentially present in terms of quantity, frequency or both as well as a rtio of differences between two or more specific modified amino acid residues and/or the enzyme itself.

A marker, compound, composition or substance is differentially present in a sample if the amount of the marker, compound, composition or substance in the sample is statistically significantly different from the amount of the marker, compound, composition or substance in another sample, or from a control value. For example, a compound is differentially present if it is present at least about 120%, at least about 130%, at least about 150%, at least about 180%, at least about 200%, at least about 300%, at least about 500%, at least about 700%, at least about 900%, or at least about 1000% greater or less than it is present in the other sample (e.g. control), or if it is detectable in one sample and not detectable in the other.

Alternatively or additionally, a marker, compound, composition or substance is differentially present between samples if the frequency of detecting the marker, etc. in samples of patients suffering from a particular disease or disorder, is statistically significantly higher or lower than in the control samples or control values obtained from healhty individuals. For example, a biomarker is differentially present between the two sets of samples if it is detected at least about 120%, at least about 130%, at least about 150%, at least about 180%, at least about 200%, at least about 300%, at least about 500%, at least about 700%, at least about 900%, or at least about 1000% more frequently or less frequently observed in one set of samples than the other set of samples. These exemplary values notwithstanding, it is expected that a skilled practitioner can determine cut-off points, etc. that represent a statistically significant difference to determine whether the marker is differentially present.

"Diagnostic" means identifying the presence or nature of a pathologic condition and includes identifying patients who are at risk of developing a specific disease or disorder. Diagnostic methods differ in their sensitivity and specificity. The "sensitivity" of a diagnostic assay is the percentage of diseased individuals who test positive (percent of "true positives"). Diseased individuals not detected by the assay are "false negatives." Subjects who are not diseased and who test negative in the assay, are termed "true negatives." The "specificity" of a diagnostic assay is 1 minus the false positive rate, where the "false positive" rate is defined as the proportion of those without the disease who test positive. While a particular diagnostic method may not provide a definitive diagnosis of a condition, it suffices if the method provides a positive indication that aids in diagnosis.

The terms "detection", "detecting" and the like, may be used in the context of detecting biomarkers, or of detecting a disease or disorder (e.g. when positive assay results are obtained). In the latter context, "detecting" and "diagnosing" are considered synonymous.

By "at risk of" is intended to mean at increased risk of, compared to a normal subject, or compared to a control group, e.g. a patient population. Thus a subject carrying a particular marker may have an increased risk for a specific disease or disorder, and be identified as needing further testing. "Increased risk" or "elevated risk" mean any statistically significant increase in the probability, e.g., that the subject has the disorder. The risk is preferably increased by at least 10%, more preferably at least 20%, and even more preferably at least 50% over the control group with which the comparison is being made.

A "test amount" of a marker refers to an amount of a marker present in a sample being tested. A test amount can be either in absolute amount (e.g., µg/ml) or a relative amount (e.g., relative intensity of signals).

A "diagnostic amount" of a marker refers to an amount of a marker in a subject's sample that is consistent with a diagnosis of a particular disease or disorder. A diagnostic amount can be either in absolute amount (e.g., µg/ml) or a relative amount (e.g., relative intensity of signals).

A "control amount" of a marker can be any amount or a range of amount which is to be compared against a test amount of a marker. For example, a control amount of a marker can be the amount of a marker in a person who does not suffer from the disease or disorder sought to be diagnosed. A control amount can be either in absolute amount (e.g., µg/ml) or a relative amount (e.g., relative intensity of signals).

The terms "polypeptide," "peptide" and "protein" are used interchangeably herein to refer to a polymer of α-amino acid residues, in particular, of naturally-occuring α-amino acids. The terms apply to amino acid polymers in which one or more amino acid residue is an analog or mimetic of a corresponding naturally-occurring amino acid, as well as to naturally-occurring amino acid polymers. Polypeptides can be modified, e.g., by the addition of carbohydrate residues to form glycoproteins, phosphorylation to form phosphoproteins, and a large number of chemical modifications (oxidation, deamidation, amidation, methylation, formylation, hydroxymethylation, guanidination, for example) as well as degraded, reduced, or crosslinked. The terms "polypeptide," "peptide" and "protein" include all unmodified and modified forms of the protein. A peptide would have a citrullinated residue or is part of the PAD enzyme.

"Detectable moiety" or a "label" refers to a composition detectable by spectroscopic, photochemical, biochemical, immunochemical, or chemical means. For example, useful labels include ³²P, ³⁵S, fluorescent dyes, electron-dense reagents, enzymes (e.g., as commonly used in an ELISA), biotin-streptavidin, dioxigenin, haptens and proteins for which antisera or monoclonal antibodies are available, or nucleic acid molecules with a sequence complementary to a target. The detectable moiety often generates a measurable signal, such as a radioactive, chromogenic, or fluorescent signal, that can be used to quantify the amount of bound detectable moiety in a sample. Quantitation of the signal is achieved by, e.g., scintillation counting, densitometry, flow cytometry, or direct analysis by mass spectrometry of intact protein or peptides (one or more peptide can be assessed) that has a potential citrullinated residue or part of the PAD enzyme. Citrullinated Arg as part of a protein or peptide can be detected directly by MS or via chemical derivatization.

"Antibody" refers to a polypeptide ligand substantially encoded by an immunoglobulin gene or immunoglobulin genes, or fragments thereof, which specifically binds and recognizes an epitope (e.g., an antigen). The recognized immunoglobulin genes include the kappa and lambda light chain constant region genes, the alpha, gamma, delta, epsilon and mu heavy chain constant region genes, and the myriad immunoglobulin variable region genes. Antibodies exist, e.g., as intact immunoglobulins or as a number of well characterized fragments produced by digestion with various peptidases. This includes, e.g., Fab' and F(ab)'₂ fragments. The term "antibody," as used herein, also includes antibody fragments either produced by the modification of whole antibodies or those synthesized de novo using recombinant DNA methodologies. It also includes polyclonal antibodies, monoclonal antibodies, chimeric antibodies, humanized antibodies, or single chain antibodies. "Fc" portion of an antibody refers to that portion of an immunoglobulin heavy chain that comprises one or more heavy chain constant region domains, CH₁, CH₂ and CH₃, but does not include the heavy chain variable region.

By "binding assay" is meant a biochemical assay wherein the biomarkers are detected by binding to an agent, such as an antibody, through which the detection process is carried out. The detection process may involve radioactive or fluorescent labels, and the like. The assay may involve immobilization of the biomarker, or may take place in solution. Further, chemical binding to the citrullinated residue can occur directly.

"Immunoassay" is an assay that uses an antibody to specifically bind an antigen (e.g., a marker). The immunoassay is characterized by the use of specific binding properties of a particular antibody to isolate, target, and/or quantify the antigen.

The phrase "specifically (or selectively) binds" to an antibody or "specifically (or selectively) immunoreactive with," when referring to a protein or peptide, refers to a binding reaction that is determinative of the presence of the protein in a heterogeneous population of proteins and other biologics. Thus, under designated immunoassay conditions, the specified antibodies bind to a particular protein at least two times the background and do not substantially bind in a significant amount to other proteins present in the sample. Specific binding to an antibody under such conditions may require an antibody that is selected for its specificity for a particular protein. A variety of immunoassay formats may be used to select antibodies specifically immunoreactive with a particular protein. For example, solid-phase ELISA immunoassays are routinely used to select antibodies specifically immunoreactive with a protein (see, e.g., Harlow & Lane, Antibodies, A Laboratory Manual (1988), for a description of immunoassay formats and conditions that can be used to determine specific immunoreactivity).

Methods for detecting citrullination" refer to the mass spectrometry (MS) base methods used to detect citrullinated peptides, polypeptides and proteins. The methods include but are not restricted to neutral loss of 1 Da when deimination occurs on Arg; neutral loss of isocyanic acid from unmodified citrulline and used this ion as a diagnostic marker for detecting protein citrullination; derivatization when chemical modification of 238Da or 239Da occurs on Cit residue (can be monitored at the peptide and protein level); enrichment of citrullinated peptides (or protiens) that is based on the specific reaction of glyoxal derivatives that is immobilized on beads/column/matrix reacts exclusively with the ureido group of the citrulline residue at low pH. As well, MS using a targeted method like multiple or selective reaction monitoring can be used to quantify the modified peptide directly. As used herein, a labeled (e.g. N15 or chemical with additional stable isotope) peptide of known concentration is added to the sample and compared directly to the endogenous (unlabeled) corresponding peptide.

The terms "subject", "patient" or "individual" generally refer to a human, although the methods of the invention are not limited to humans, and should be useful in other animals (e.g. birds, reptiles, amphibians, mammals), particularly in mammals, since albumin is homologous among species.

"Sample" is used herein in its broadest sense. A sample may comprise a bodily fluid including blood, serum, plasma, tears, aqueous and vitreous humor, spinal fluid; a soluble fraction of a cell or tissue preparation, or media in which cells were grown; or membrane isolated or extracted from a cell or tissue; polypeptides, or peptides in solution or bound to a substrate; a cell; a tissue; a tissue print; a fingerprint, skin or hair; fragments and derivatives thereof. Subject samples usually comprise derivatives of blood products, including blood, plasma and serum.

The term "modulation of specific PAD isoforms" include, but are not limited to, increasing or decreasing the activity of endogenous PAD isoforms using gene therapy, siRNA, known inhibitors of PADs, or site-directed mutagenesis.

### EXAMPLES

Tissue Samples: Human Left ventricular (LV) transmural tissue samples were obtained from patients with end-stage ISHD, IDCM and non-failing donor hearts. The tissue from these deidentified tissue banked samples was collected in cardioplegic solution and stored in liquid nitrogen. The samples were provided to us by Dr. Cris Dos Remoidois, University of Sidney, Australia. A subset is analyzed in this study (Table 2).

| | TABLE 2 Characteristic of HF Samples | | | | |
|---|---|---|---|---|---|
| Code # | Type | ID# | Sex | Age | LVEF (%) |
| | | | | | |
| 1 | ISHD | 3.062 | M | 31 | 23 |
| 2 | ISHD | 3.007 | M | 45 | - |
| 3 | ISHD | 2.063 | M | 46 | 25 |
| 4 | ISHD | 3.123 | M | 47 | 20 |
| 5 | ISHD | 3.078 | M | 50 | - |
| 6 | ISHD | 3.106 | M | 52 | - |
| 7 | ISHD | 3.105 | M | 54 | - |
| 8 | ISHD | 3.143 | M | 54 | - |
| 9 | ISHD | 3.075 | F | 43 | - |
| 10 | ISHD | 3.103 | F | 49 | - |
| 11 | IDCM | 2.092 | M | 43 | - |
| 12 | IDCM | 3.104 | M | 46 | - |
| 13 | IDCM | 3.138 | M | 56 | 15 |
| 14 | IDCM | 2.095 | M | 57 | 10 |
| 15 | IDCM | 4.098 | M | 58 | 20 |
| 16 | IDCM | 4.058 | M | 60 | 15 |
| 17 | IDCM | 3.096 | F | 23 | 15 |
| 18 | IDCM | 3.159 | F | 31 | 20 |
| 19 | IDCM | 2.111 | F | 53 | 20 |
| 20 | IDCM | 2.115 | F | 54 | 22 |
| 21 | Donor | 4.015 | M | 19 | - |
| 22 | Donor | 2.152 | M | 23 | - |
| 23 | Donor | 4.013 | M | 23 | - |
| 24 | Donor | 3.135 | M | 26 | - |
| 25 | Donor | 3.145 | M | 39 | - |
| 26 | Donor | 2.149 | M | 44 | - |
| 27 | Donor | 3.141 | M | 52 | - |
| 28 | Donor | 3.084 | F | 27 | - |
| 29 | Donor | 3.073 | F | 41 | - |
| 30 | Donor | 3.056 | F | 45 | - |

Subfractionation of Heart Tissue: The method produces three fractions based on solubility at different pHs: (1) cytoplasmic-enriched extract (neutral pH), (2) myofilament-enriched extract (acidic pH), and (3) membrane protein-enriched pellet. Fractionation of heart tissue in this manner provides the basis for in-depth proteomic analysis (Kane et al., Cardiovascular Proteomics 2007, 357:87-90).

Targeted analysis of protein citrullination by mass spectrometry: MS has become the method of choice for the analysis of PTMs on proteins and peptides. However, PTMs are typically present in relatively small amounts in heterogeneous and complex protein mixtures. Specifically, in the case of citrullination, the identification is complicated by the fact that the mass shift resulting from the conversion of arginine to citrulline is small (+1 Da), making it difficult to identify the citrullination using low-resolution MS instrumentation. To overcome this challenge, we used an LTQ Orbitrap high-resolution MS (Stensland et al., Rapid Commun Mass Spectrom 2009, 23:2754-2762). Also, as this derivatization strategy adds a specific mass tag of ±239 Da (Figure 1A) to the citrulline reside, we are able to confidently identify the site of modification. Our recent investigations show that by using this strategy, we can identify citrullinated peptides from a heterogeneous mixture such as a tryptic digest of bovine serum albumin (BSA) (Figure 1B).

Two-dimensional gel electrophoresis: Two-dimensional gel electrophoresis (2DE) was performed on immobilized pH gradient 18-cm strips (GE Healthcare, Buckinghamshire, UK), pH ranges 4 to 7 in the first dimension. The sample was re-suspended in 2-DE lysis buffer containing 4% w/v CHAPS, 7 M urea, 2 M thiourea, 10 mM Tris-HCl, pH 8.3 and 1 mM EDTA. Before performing 2D-DIGE, protein samples were labeled with N-hydroxy succinimidyl ester-derivatives of the cyanine dyes Cy2, Cy3 and Cy5. Briefly, 150 µg of protein sample was minimally labeled with 375 pmol of either Cy3 or Cy5 for comparison on the same 2-DE. To facilitate image matching and cross-gel statistical comparison, a pool of all samples was also prepared and labeled with Cy2 at a molar ratio of 2.5 pmol Cy2 per µg of protein as an internal standard for all gels. Thus, the triplicate samples and the internal standard could be run and quantify on multiple 2-DE. The labeling reactions were performed in the dark on ice for 30 min and then quenched with a 20-fold molar ratio excess of free L-lysine for 10 min. The differentially Cy3- and Cy5-labeled samples were then mixed with the Cy2-labeled internal standard and reduced with dithiothreitol for 30 min. IPG buffer, pH 4-7 nonlinear (2% (v/v), GE Healthcare) was added and the final volume was adjusted to 350 µl with 2D-lysis buffer for rehydration. Immobilized pH gradient (IPG) Strips (18 cm pH 4-7 linear gradients) were actively rehydrated with the sample (150µg of protein in 350 µL IEF buffer) at 50 V for 12 hrs, followed by a rapid voltage ramping consisting of 1 hr each at 250, 500, and 1000 V, followed by 10000 V for 45 kVh at 20°C. Isoelectric focusing was performed a total of 62.5 kV-h at 20°C. Strips were equilibrated in 6 M urea, 30% (v/v) glycerol, 4% SDS (w/v), 100 mM Tris-HCl (pH8.8), 65 mM dithiothreitol for 20 min and then in the same buffer containing 240 mM iodoacetamide for another 20 min. The equilibrated IPG strips were transferred onto 20 x 20-cm 10% polyacrylamide gels. Gels were run overnight on a Protean® II XL system (Bio-Rad) at 90 V with 2(n-morpholino) ethansulfonic acid (MES) running buffer. Gels were subsequently scanned using a Typhoon variable mode imager 9210 (GE Healthcare). The majority of spots visible with CyDye staining are usually visible with silver staining, according to the protocol of Shevchenko et al. (Anal Chem 1996, 68:850-858).

In-gel digestion and peptide extraction: Upon completion of gel staining, individual bands were excised from the gel. In-gel digestion and peptide extraction was achieved using the improved protocol previously (Zhang et al., J Proteome Res 2007, 6:2295-2303). Briefly, cTnI bands were excised, cut into 1 mm³ pieces, and washed 3 times with 50% acetonitrile/25 mM ammonium bicarbonate for 15 min with shaking. Gel pieces were incubated with 25 mM ammonium bicarbonate +10 mM dithiothreitol for 60 min at 55 °C, washed with acetonitrile (ACN), then incubated with 20 mM ammonium bicarbonate + 55 mM iodoacetamide (freshly made) for 30 minutes in the dark. The gel pieces were washed with acetonitrile, air dried, and rehydrated with 12.5 ng/µL trypsin (Promega, sequencing grade, Madison, WI) in 25mM ammonium bicarbonate, then incubated at 37 °C for 18 hr or overnight. The liquid was transferred to a clean tube. Peptides were extracted twice using 50% ACN+0.1 TFA% for 20 min at 25 °C, followed by 20 min of shaking, 1 min of centrifugation, and combined two times of extraction with the liquid from the previous step.

Immunoblotting analysis: Protein samples were dissolved in Laemmli buffer (50 mM TrisHCl, pH 6.8, 2% SDS, 10% glycerol, bromophenol blue) with 5% β-mercapto-ethanol and incubated at 95°C for five minutes. The samples were loaded on a 10% TrisHCl polyacrylamidegel (Biorad, Hercules, CA, USA) and electrophoresis was performed by applying 150 V for 70 minutes. After SDS-PAGE, separated proteins from mouse were blotted to nitrocellulose membrane (Millipore,USA). The transfer was carried out in a buffer containing 25 mM Tris, 192 mM glycine and 10% volume fraction of methanol, pH=8.3 at a constant voltage setting of 100 V for 60 min in a cell for electrophoretic transfer. The presence of citrullinated proteins on the nitrocellulose blots was detected using the anti-modified citrulline (AMC) detection kit (Upstate, Charlottesville, VA, USA) according to the manufacturer's protocol.

Detection of citrullinated protein *in situ:* Immunostaining of citrullinated proteins was performed by using anti-modified citrulline IgG polyclonal antibody. Briefly, slides for citrullination staining were modified in a strong acid solution containing antipyrine and 2,3-butanedoine for 3 hours at 37° C. Endogenous peroxidase activity was blocked by incubation in 0.3% H₂O₂ in methanol for 18 minutes. Non-specific protein activity was blocked by incubation with a non-serum protein solution (DAKO Corporation, Carpinteria, CA). Scoring for the citrulline staining was also performed blinded using 5 point scale (1-3 in 0.5 increments) corresponding to minimal, moderate and marked citrullination stains.

Results: Our data indicate that citrullination occurs in the myocardium. We have detected citrullinated protein in healthy like diseases samples. Similar, we found modified protein in each exanimate fractions. Interesting we have found a few citrullinated residue in myosin and tropomyosin which are the molecular motor that drives sarcomere shortening through a cyclic, ratchet-like interaction with actin. Therefore, it appears that citrullination of myocardial proteins may influence cardiac performance in heart failure.

*In vivo* targeted analysis of protein citrullination by tandem mass spectrometry: We investigated the 2,3-butanedione and antipyrine modification for specific detection and identification of citrulline-containing peptides by tandem MS/MS. Under the conditions applied, the modification by 2,3-butanedione/antipyrine is absolutely specific for citrulline. Under this condition we were able to identify several proteins in untreated subcellular fractions of human myocardium (Tables 1A and 1B). Interestingly, some modified sites seems to be more specific for HF vs. control (Figure 1).

Citrullination occurs in human heart tissue: To support our hypothesis, we have examined human heart tissue for the presence of citrullinated proteins. Using a commercially available antibody (Anti-Citruline (modified) Detection Kit, Millipore) to citrullinated proteins, we have verified that citrullinated proteins are present in human heart tissue (Figure 2a). Furthermore we use immunohistochemistry to localize citrullination in heart tissue (Figure 2b). As shown in Fig. 2a two main proteins, myosin and actin act differently in compared groups. In HF tissue, a significant increase of myosin heavy chain citrullination vs. control was seen. On the other hand actin showed opposite pattern to myosin. Additional the IHC staining of control heart tissue proved occurrence of citrullinated proteins in human heart tissue.

DIGE and mass spectrometry: DIGE images of the nine gels enabled localization of variation spots. Spots detected by CyDyes staining were excised and subjected for identification. In total, up to 25 different spots were detected on the nine gels. Orbi trap LTQ was used to analyze the peptides after in-gel digestion of each spot. A total of 25 protein spots, only 7 were successfully identified. Table 4 shows the proteins which were identified.

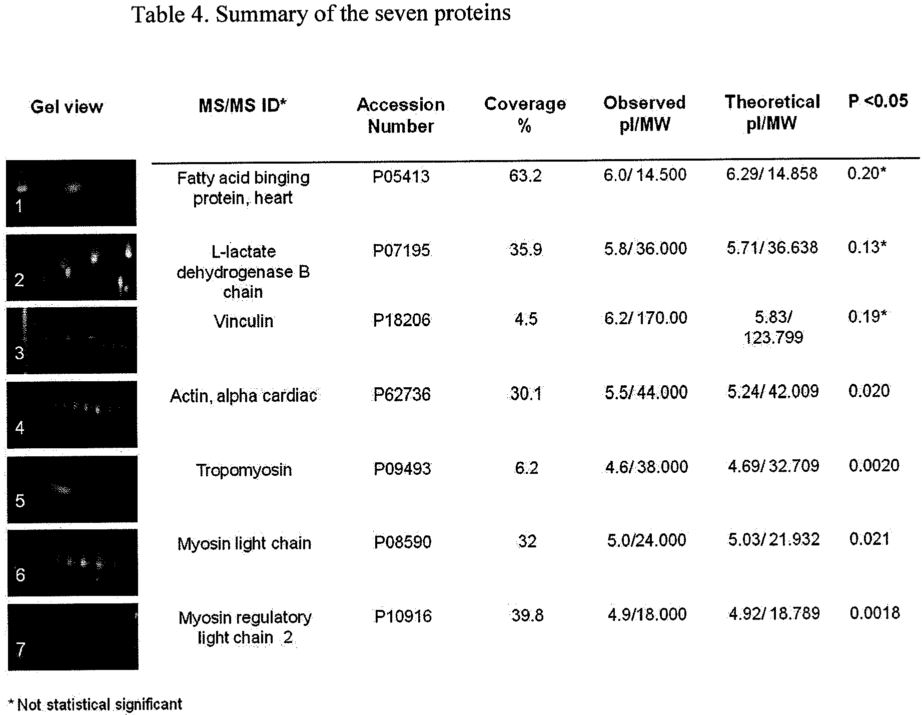

## Claims

1. A method of diagnosing myocardial disease or ischemic heart disease in a subject, comprising detecting the presence of a citrullinated protein in a biological sample obtained from a subject, wherein the level of citrullinated protein is indicative of myocardial disease or ischemic heart disease,
wherein the biological sample is a myocardial tissue biopsy, and wherein the citrullinated protein is myosin heavy chain or actin.

2. A method of diagnosing heart failure in a subject, comprising detecting the presence of a citrullinated protein in a biological sample obtained from a subject, wherein the level of citrullinated protein is indicative of heart failure,
wherein the biological sample is a myocardial tissue biopsy, and wherein the citrullinated protein comprises an amino acid sequence selected from the group consisting of SEQ ID NO:2, SEQ ID NO:3, SEQ ID NO:4, SEQ ID NO:6, SEQ ID NO:8, SEQ SEQ ID NO:10, SEQ ID NO:12, SEQ ID NO:14, SEQ ID NO:15, SEQ ID NO:16 and SEQ ID NO:18 or
selected from the group consisting of SEQ ID NO:20, SEQ ID NO:21, SEQ ID NO:24, SEQ ID NO:27, SEQ ID NO:28, SEQ ID NO:29, SEQ ID NO:31, SEQ ID NO:33, and SEQ ID NO:34

3. The method of claims 1 or 2, wherein the citrullinated protein is elevated or decreased compared to the control amount of the citrullinated protein.

4. The method of claims 1 to 3, wherein the citrullinated protein comprises the post-translational conversion of an arginine residue to citrulline.

5. The method of claims 1 to 4, wherein the presence of the citrullinated protein is detected using mass spectrometry, high resolution mass spectrometry, tandem mass spectrometry, binding assay, immunoassay, antibody binding or immunohistochemistry.

## Patentansprüche

1. Verfahren zum Diagnostizieren einer Myokarderkrankung oder ischämischen Herzerkrankung bei einem Probanden, umfassend das Nachweisen der Anwesenheit eines citrullinierten Proteins in einer biologischen Probe, die einem Probanden entnommen wurde, wobei die Konzentration des citrullinierten Proteins eine Myokarderkrankung oder ischämische Herzerkrankung anzeigt;
wobei die biologische Probe ein Myokardgewebebioptat ist; und
wobei es sich bei dem citrullinierten Protein um eine schwere Kette von Myosin oder um Actin handelt.

2. Verfahren zum Diagnostizieren einer Herzinsuffizienz bei einem Probanden, umfassend das Nachweisen der Anwesenheit eines citrullinierten Proteins in einer biologischen Probe, die von einem Probanden erhalten wurde, wobei die Konzentration des citrullinierten Proteins eine Herzinsuffizienz anzeigt;
wobei die biologische Probe ein Myokardgewebebioptat ist; und
wobei das citrullinierte Protein eine Aminosäuresequenz umfasst, die aus der Gruppe ausgewählt ist, die aus SEQ ID Nr. 2, SEQ ID Nr. 3, SEQ ID Nr. 4, SEQ ID Nr. 6, SEQ ID Nr. 8, SEQ ID Nr. 10, SEQ ID Nr. 12, SEQ ID Nr. 14, SEQ ID Nr. 15, SEQ ID Nr. 16 und SEQ ID Nr. 18 besteht, oder aus der Gruppe ausgewählt ist, die aus SEQ ID Nr. 20, SEQ ID Nr. 21, SEQ ID Nr. 24, SEQ ID Nr. 27, SEQ ID Nr. 28, SEQ ID Nr. 29, SEQ ID Nr. 31, SEQ ID Nr. 33 und SEQ ID Nr. 34 besteht.

3. Verfahren gemäß Anspruch 1 oder 2, wobei das citrullinierte Protein im Vergleich zur Kontrollmenge des citrullinierten Proteins erhöht oder reduziert ist.

4. Verfahren gemäß Anspruch 1 bis 3, wobei das citrullinierte Protein die posttranslationale Umwandlung eines Argininrests in Citrullin umfasst.

5. Verfahren gemäß Anspruch 1 bis 4, wobei die Anwesenheit des citrullinierten Proteins mit Hilfe von Massenspektrometrie, hochauflösender Massenspektrometrie, Tandem-Massenspektrometrie, Bindungsassay, Immunassay, Antikörperbindung oder Immunhistochemie nachgewiesen wird.

## Revendications

1. Méthode de diagnostic d'une maladie myocardique ou d'une cardiopathie ischémique chez un sujet, comprenant la détection de la présence d'une protéine citrullinée dans un échantillon biologique obtenu à partir d'un sujet, dans laquelle le taux de protéine citrullinée est indicatif d'une maladie myocardique ou d'une cardiopathie ischémique,
dans laquelle l'échantillon biologique est une biopsie de tissu myocardique, et
dans laquelle la protéine citrullinée est la chaîne lourde de myosine ou l'actine.

2. Méthode de diagnostic d'une insuffisance cardiaque chez un sujet, comprenant la détection de la présence d'une protéine citrullinée dans un échantillon biologique obtenu à partir d'un sujet, dans laquelle le taux de protéine citrullinée est indicatif d'une insuffisance cardiaque,
dans laquelle l'échantillon biologique est une biopsie de tissu myocardique, et
dans laquelle la protéine citrullinée comprend une séquence d'acides aminés choisie dans le groupe consistant en SEQ ID NO : 2, SEQ ID NO : 3, SEQ ID NO : 4, SEQ ID NO : 6, SEQ ID NO : 8, SEQ ID NO : 10, SEQ ID NO : 12, SEQ ID NO : 14, SEQ ID NO : 15, SEQ ID NO : 16 et SEQ ID NO : 18, ou
choisie dans le groupe consistant en SEQ ID NO : 20, SEQ ID NO : 21, SEQ ID NO : 24, SEQ ID NO : 27, SEQ ID NO : 28, SEQ ID NO : 29, SEQ ID NO : 31, SEQ ID NO : 33, et SEQ ID NO : 34.

3. Méthode selon les revendications 1 ou 2, dans laquelle la protéine citrullinée est augmentée ou diminuée en comparaison à la quantité de témoin de la protéine citrullinée.

4. Méthode selon les revendications 1 à 3, dans laquelle la protéine citrullinée comprend la conversion post-traductionnelle d'un résidu d'arginine en citrulline.

5. Méthode selon les revendications 1 à 4, dans laquelle la présence de la protéine citrullinée est détectée à l'aide d'une spectrométrie de masse, d'une spectrométrie de masse haute résolution, d'une spectrométrie de masse en tandem, d'un dosage de liaison, d'un immunoessai, d'une liaison à l'anticorps ou d'une immunohistochimie.
